# EUROPEAN PATENT SPECIFICATION

(11) **EP 3 666 341 B1**
(45) Date of publication and mention of the grant of the patent: **23.03.2022**
(21) Application number: 18212580.7
(22) Date of filing: 14.12.2018
(51) Int. Cl.: A61Q 19/00, A61K 9/70, A61L 15/00, A61K 35/747, A61P 17/00

(54) **TOPICAL FORMULATION IN FORM OF A PATCH, A BANDAGE OR A PLASTER COMPRISING PROBIOTIC BACTERIA, AND USE THEREOF IN A METHOD FOR TREATING OR PREVENTING SKIN DISORDERS**
TOPISCHE FORMULIERUNG IN FORM EINES PFLASTERS, VERBAND ODER PFLASTER MIT PROBIOTISCHEN BAKTERIEN UND VERWENDUNG DAVON IN EINEM VERFAHREN ZUR BEHANDLUNG ODER VORBEUGUNG VON HAUTERKRANKUNGEN
FORMULATION TOPIQUE SOUS FORME DE PATCH, DE BANDAGE OU DE PANSEMENT COMPRENANT DES BACTÉRIES PROBIOTIQUES ET SON UTILISATION DANS UN PROCÉDÉ POUR LE TRAITEMENT OU LA PRÉVENTION DE TROUBLES CUTANÉS

(43) Date of publication of application: 17.06.2020
(73) Proprietor: DWI - Leibniz-Institut für Interaktive Materialien e.V., 52074 Aachen (DE)
(72) Inventor: Heine, Elisabeth, 52385 Nideggen (DE); Lütticken, Rudolf, 52072 Aachen (DE); Gartzen, Rita, 52146 Würselen (DE); Khalfallah, Ghazi, 52070 Aachen (DE)
(74) Representative: Gille Hrabal Partnerschaftsgesellschaft mbB Patentanwälte

(56) References cited:
- WO-A1-2017/220525
- US-B2- 8 685 389
- MICHAEL N PASTORE ET AL: "Transdermal patches: history, development and pharmacology : History of transdermal patches", BRITISH JOURNAL OF PHARMACOLOGY, vol. 172, no. 9, 18 March 2015 (2015-03-18), pages 2179-2209, XP055585122, UK ISSN: 0007-1188, DOI: 10.1111/bph.13059

### SEQUENCE LISTING

<110> DWI Leibniz-Institute for Interactive Materials e. V.
<120> Topical formulation in form of a patch, a bandage or a plaster comprising probiotic bacteria, and use thereof in a method for treating or preventing skin disorders
<130> Prob_V1 /T68194
<160> 1
<170> PatentIn version 3.5
<210> 1
   <211> 895
   <212> DNA
   <213> Lactobacillus plantarum 8P-A3 (DSM 32835)
<220>
   <221> 16S rRNA gene
   <222> (1)..(895)
<400> 1
   cggatgctta atgcgttagc tgcagcactg aagggcggaa accctccaac acttagcatt        60
   catcgtttac ggtatggact accagggtat ctaatcctgt ttgctaccca tactttcgag       120
   cctcagcgtc agttacagac cagacagccg ccttcgccac tggtgttctt ccatatatct       180
   acgcatttca ccgctacaca tggagttcca ctgtcctctt ctgcactcaa gtttcccagt       240
   ttccgatgca cttcttcggt tgagccgaag gctttcacat cagacttaaa aaaccgcctg       300
   cgctcgcttt acgcccaata aatccggaca acgcttgcca cctacgtatt accgcggctg       360
   ctggcacgta gttagccgtg gctttctggt taaataccgt caatacctga acagttactc       420
   tcagatatgt tcttctttaa caacagagtt ttacgagccg aaacccttct tcactcacgc       480
   ggcgttgctc catcagactt tcgtccattg tggaagattc cctactgctg cctcccgtag       540
   gagtttgggc cgtgtctcag tcccaatgtg gccgattacc ctctcaggtc ggctacgtat       600
   cattgccatg gtgagccgtt accccaccat ctagctaata cgccgcggga ccatccaaaa       660
   gtgatagccg aagccatctt tcaagctcgg accatgcggt ccaagttgtt atgcggtatt       720
   agcatctgtt tccaggtgtt atcccccgct tctgggcagg tttcccacgt gttactcacc       780
   agttcgccac tcactcaaat gtaaatcatg atgcaagcac caatcaatac cagagttcgt       840
   tcgacttgca tgtattaggc acgccgccag cgttcgtcct gagccagaat caaac            895

## Description

### Technical Field

This invention relates to pharmaceutical topical formulations comprising beneficial or probiotic bacteria ("probiotics"), preferably belonging to the order *Lactobacillales,* having antimicrobial activity against skin pathogens, which exert a curing effect on skin disorders, and to their use in a method of treating or preventing skin disorders.

In particular, the invention relates to pharmaceutical topical formulations intended for treatment of skin disorders caused or triggered by bacteria or for preventing an aggravation of those disorders. The invention is primarily, but not exclusively, intended to treat acne and infections associated with burn wounds, venous leg ulcers or other skin lesions.

### Background Art

Microorganisms physiologically permanently present on certain areas of the skin, mostly "non-pathogenic" microorganisms or opportunists, influence skin disorders in a way that they contribute to the further development, e. g. inflammation or tissue destruction, leading to overt acute or chronic skin infections.

Primary causes for skin lesions might be hormonal changes like in acne vulgaris (Beylot, C. et al., 2014), venous insufficiency as in venous leg ulcers, atopic dermatitis or burns.

Skin infections can be triggered or caused by a variety of opportunistic or obligate pathogens, e. g. *Staphylococcus* species, in particular *S*. *aureus, Streptococcus* species, *Enterobacteriaceae, Pseudomonas aeruginosa* or fungi. In acne vulgaris, a frequent skin disease mainly of adolescents and young adults, there are indications that the anaerobic bacterium *Cutibacterium acnes* (formerly *Propionibacterium acnes*) is co-causing the symptoms (Beylot, C. et al., a.a.O.).

In these skin disorders, antibiotics are often applied as primary or supporting therapy, either topically or systemically. The application of antibiotics can cause biological side-effects like suppression of the physiological (beneficial) microbial flora on the skin thereby allowing pathogenic microorganisms like yeasts and other fungi to overgrow and cause an infection.

Another unwanted secondary effect of the use of antibiotics is the increasingly problematic development of antibiotic resistances to such an extent that, especially in hospitals, no effective antibiotic for treatment of serious infections may be available (Bassetti, M. et al., 2017). Thus, alternative ways for treatment of skin disorders, attributable to the action of microorganisms, avoiding the use of antibiotics or at least reducing the length of antibiotic therapy are much-needed.

There is a constant demand for new therapeutic means for treating infectious skin disorders avoiding these disadvantages, in particular suppression of the physiological microbial flora on the skin and development of resistances.

Probiotic bacteria (probiotics) have been defined by a FAO/WHO expert consultation as "live microorganisms which when administered in adequate amounts confer a health benefit on the host". (FAO/WHO, 2002).

Probiotic bacteria have been proposed in the past for topical treatment of the skin and for treatment of skin disorders, respectively, but they were *directly* applied onto the skin.

WO 2017/220525 A1 discloses the direct topical application of selected *Lactobacillus* strains, in particular *L. plantarum, L. pentosus* and/or *L. rhamnosus,* against common skin pathogens. Lactic acid produced by the strains is considered an important antimicrobial factor. Effects of lactobacilli discussed in this document are i) to preserve a healthy skin pH (+/- 5.5), mainly by producing lactic acid, ii) production of antimicrobial compounds and competitive exclusions of pathogens, iii) modulation of immune response and iv) strengthening of the epithelial barrier. The formulations disclosed are topical skin compositions, in particular in form of a gel, cream, foam, lotion or ointment. Specific skin disorders treated in the Examples are acne vulgaris and tinea pedis.

EP 1 863 899 B1 corresponding to WO 2006/104403 A1 discloses topical compositions comprising a specific *Micrococcus luteus* strain for controlling skin disorders caused by bacteria, such as impetigo, erysipelas, folliculitis, acne, boils, carbuncles, cellulitis, pitted keratolysis, intertrigo, trichomycosis, mastitis, toe infections such as tinea, and body odour. The topical formulations comprise the microorganisms and suitable carriers and excipients and are in the form of cream, gel, emulsion, oil, paste, lotion, wash, suspension, spray (including nasal spray), powder, stick, roll-on or aerosol, face wash, soap, cream, unguent, gel. Transdermally administrable formulations are mentioned on p. 10, I. 25. In this case, the *M. luteus* formulations are administered indirectly such as in material for contacting the skin or mucosa. As examples for such indirect administration forms, nappies, wet wipes, sanitary pads and clothing articles are mentioned. The *M. luteus* can be applied to the material by known techniques such as spraying and drying according to the document.

WO 2011/070509 A1 is related to the use of probiotic microorganisms for preventing or treating impairments of the skin's microrelief. The surface of the skin is not smooth, but has a relief of fine lines, different from wrinkles. These fine lines can be seen with a magnifying glass in the case of children and with the naked eye in the case of the elderly. The expression "microrelief" designates structures of polygonal shapes formed by fine lines or grooves which cross each other. In particular, *Lactobacillus sp.* are disclosed, namely *Lactobacillus casei* and a specific strain thereof, *L. johnsonii, L. acidophilus, L. reuteri, L. paracasei* as well as *Bifidobacterium sp., cocci,* yeasts and sporulating bacteria. *Lactobacillus plantarum* is mentioned besides other *Lactobacillus* species. Preferred microorganisms, according to this document, produce lactic acid by fermentation of sugar. The compositions can be applied topically, orally or parenterally. Topical formulations are ointments, creams, milks, pomades, powders, impregnated pads, solutions, gels, sprays, lotions or suspensions. They may also be in the form of microspheres or nanospheres or lipid or polymer vesicles or polymer patches and hydrogels allowing controlled release. Transdermal systems for active or passive release of active agents are also mentioned, for example a patch or gel patch (hydrogel).

US 2017/0157034 A1 discloses a topical stick formulation comprising probiotic bacteria for hydrating the skin, reducing and preventing fine lines and wrinkles, treating acne and decreasing skin inflammation. Bacteria specifically mentioned are *Streptococcus thermophilus, Streptococcus salivarius* subspecies *thermophilus* strain 1131, *Bifidobacterium longum, Lactobacillus paracasei* and *Streptococcus salivarius.* The formulation (i.e. the stick comprising the bacteria and excipients which has been solidified) is applied topically in a thin layer to the entire affected area, e.g. the skin of face, back or chest. The stick is preferably rubbed directly onto a blemish.

WO 2006/136420 A2 discloses the topical use of microorganisms of the genus of *Lactobacillus,* in particular *L. paracasei, L. brevis, L. fermentum, L. buchneri* or *L. delbrückii* for protecting the skin against pathogenic microorganisms. The formulations can be in form of a paste, an ointment, a lotion, a cream, a gel or a transdermal patch. The formulations are useful for treating dermatitis, e.g. atopic dermatitis, psoriasis, poison ivy dermatitis, eczema herpeticum, kerion or scabies.

WO 2013/188626 A2 describes probiotic-containing compositions comprising a probiotic, an oxygen-carrying compound and a bio-compatible binder. They are suitable for incorporation in aqueous personal care formulations such as cosmetic and non-cosmetic lotions, soaps, creams, shampoos, body washes and the like. Numerous microorganisms are described to be suitable, inter alia a large number of *Lactobacilli* including *L. plantarum.* The formulation may also be in the form of microspheres. The effect of probiotics mentioned in this document is a health benefit when topically applied or orally administered in adequate amounts.

WO 2010/013182 A1 discloses the use of a probiotic microorganism for treating and/or preventing dandruff disorders of the scalp and cosmetic preparations comprising probiotics. *Lactobacillus sp.,* i.e. *L. paracasei* ST11, and *Bifidobacterium sp.* are disclosed specifically as an agent for preventing and/or treating scalp disorders and in particular dandruff conditions of the scalp. Further, treatment of inflammation of the scalp, dryness or xerosis of the scalp, pruritus of the scalp, or seborrhoeic dermatitis of the scalp can be treated with the formulations according to this document as well as skin infections of the scalp by *Malassezia* sp.. The application can be performed orally or topically. Other *Lactobacilli* are also mentioned, inter alia *L. plantarum.* The topical formulations can be scalp care gel, scalp care milk, scalp care cream, hair care gel, or two-part-compositions comprising e.g. a scalp lotion and an antidandruff shampoo, or a scalp care cream and an antidandruff lotion.

WO 2011/029784 A1 corresponding to EP 2 477 637 B1 discloses a probiotic *L. rhamnosus* strain and topical and oral uses thereof. The applications comprise skin inflammatory and/or allergic skin affections, such as eczema, acne, seborrheic and atopic dermatitis, and sensitive skin. The formulations can be applied topically such as in form of pastes, lotions, solutions, gels, fluids for the body, creams, solar creams, anti-age creams, ointments, emulsions, lather, liniment, powder, aqueous suspension, oily suspension or solution or biphasic suspension or solution, make-up products such as foundation, blusher, powder, eye-shadow, mascara, eyeliner, lip liner, shampoos, bath foams, serums, make-up removers.

The strain *L. plantarum* 8P-A3 is known and has been widely used since 1973 in the Russian pharmaceutical market in several probiotic products such as "Lactobacterinum", "Biovestin-lacto", "Lactonorm", "Florin-forte" and others (A. Tsapieva, 2011). These products, according to the document, are effective for treating diarrhoea of different aetiology, dysbiosis conditions, bacterial vaginosis, acute enteric infection in children, the combined therapy of *Helicobacter pylori-related* diseases and others. Also, the ultrafiltrate of the culture medium is reported to have been used as a medicine. The strain 8P-A3 used by Tsapieva et al. (2011) has been isolated from "Lactobacterinum siccum" (Microgen, Moscow, Russia). The strain was determined as gram-positive bacilli. It demonstrated a very high capability to prevent the growth of all indicator pathogenic bacteria used, i.e. *Streptococcus pyogenes, Streptococcus agalactiae, Staphylococcus aureus, Enterococcus faecalis, Escherichia coli, Pseudomonas aeruginosa, Klebsiella oxytoca, and Klebsiella pneumoniae.*

RU 2 224 018 C discloses the waste-free preparation of probiotics using *Lactobacillus* 8PA3, wherein a bacterial suspension of 8P-A3 grown in a culture medium is subjected to ultrafiltration using a separation device using hollow fibers VPU-15 having a cut-off of 15 kDa or on membranes made form cellulose triacetate 145 or polysulfone 146. The concentrate of living bacteria is used for the production of various dosage forms of probiotics, whereas the cell-free ultrafiltrate is hermetically sealed in vials and then stabilized by heat treatment at 80-110 °C for 15 to 30 min. The thus-obtained ultrafiltrate is reported to be stable for at least two years and is used as biological stimulator. The mode of application of the ultrafiltrate is not disclosed.

US 8 685 389 B2 discloses a method for treating skin infections caused by methycillin-sensitive *Staphylococcus aureus* by topical application of a pharmaceutical composition comprising 0.5 % to 1% by weight of *Lactobacillus johnsonii* CNCM I-1225 to the infected skin. The compositions are in form of salves, creams, milks, ointments, powders, soaked swabs, solutions, gels, lotions or suspensions. They can also be in form of microspheres or nanospheres, or lipid or polymeric vesicles, or of polymer patches and of hydrogels, which allow controlled release.

M.N. Pastore et al., "Transdermal patches: history, development and pharmacology", British Journal of Pharmacology 2015, 172, p. 2179 to 2209 is a review summarizing the technolgy of transdermal patches for drug delivery.

The object of the present invention is to provide pharmaceutical formulations, in particular pharmaceutical and cosmetic topical formulations, for beneficially influencing infectious skin disorders without the use of antibiotics.

The invention further encompasses the use of specific beneficial microbes (probiotic bacteria) in form of a specific topical formulation, in particular a plaster, bandage or patch, in a method for treating skin disorders.

The invention further provides a cosmetic use of said plasters or patches, preferably for cosmetically treating acne.

In the present invention the probiotic bacteria do not directly reside or multiply on the skin but deploy their healing effect through membranes.

By not directly applying the active probiotics - in contrast to a direct application of the probiotics onto the skin - the invention assures that the probiotic bacteria cannot enter skin lesions. This might be important when treating a skin disorder in patients with an (yet unknown) immunodeficiency (e.g. neutropenia); in such persons, bacteria belonging to the greening (viridans) Streptococci (which include probiotic species) are among the commonest causes of sepsis (Tunkel, A. R. and Sepkowitz, K. A., 2002).

### Citation list (non-patent literature)

Beylot, C. et al. (2014) Propionibacterium acnes: an update on its role in the pathogenesis of acne. Journal of the European Academy of Dermatology and Venereology : JEADV 28 (3):271-8. doi:10.1111/jdv.12224
Bassetti, M. et al. (2017) Antimicrobial resistance in the next 30 years, humankind, bugs and drugs: a visionary approach. Intensive Care Med 43 (10):1464-75. doi:10.1007/s00134-017-4878-x
Tunkel, A. R., Sepkowitz, K. A. (2002) Infections caused by viridans streptococci in patients with neutropenia. Clin Infect Dis 34 (11):1524-9. doi:10.1086/340402
Tsapieva, A. et al. (2011) Structure of plantaricin locus of Lactobacillus plantarum 8P-A3. Beneficial microbes 2 (4):255-61. doi:10.3920/BM2011.0030
Food and Agriculture Organization of the United Nations/World Health Organisation (FAO/WHO), 2002, Joint FAO/WHO Working Group on Drafting Guidelines for the Evaluation of Probiotics in Food, London Ontario, Canada. Available at http://www.who.int/foodsafety/fs_management/en/probiotic_guidelines.pdf.

### Description of the invention

In the first aspect, the invention provides a topical pharmaceutical formulation comprising a pure culture of a probiotic bacterial strain or a mixed culture of probiotic bacteria (preferably of the order *Lactobacillales*) sandwiched between two membranes to be topically applied to diseased skin. The pouch may be part of a plaster or patch which can be applied to the diseased skin.

The invention provides a formulation comprising two membranes, one of them being semipermeable, which allows water and other substances from the skin to diffuse into the pouch and metabolic products of the enclosed probiotic bacteria to diffuse through it and to thereby get in contact with the diseased skin. The semipermeable membrane does not allow the enclosed probiotics to permeate through the membrane pores. The second membrane may be chemically identical to the first membrane, but it is water-impermeable and thus protects the probiotic from contact with the surroundings.

The semipermeable membrane of the composition is required to have a pore size which does not allow penetration of the bacteria. Consequently, a pore size smaller than the size of the enclosed bacteria is suitable. That is, the semipermeable membrane is any membrane suitable for microfiltration. Therefore, the semipermeable membrane preferably has a pore size of at least 0.1 µm, preferably 0.1 µm to 0.4 µm, and more preferably 0.15 to 0.25 µm. According to the invention, the water-impermeable membrane is defined to have no pores in the sense that it does neither allow penetration of water molecules in liquid state nor of larger molecules.

Shape and size of pores in membranes largely depend from the manufacturing process and can generally be specified only with difficulties. Therefore, the pore size for membranes is usually determined by test filtrations, and the pore size is designated as the diameter of the smallest particle which did not pass the membrane. That is, the pore size is determined indirectly and is merely a cut-off size.

Any commercially available semipermeable membrane is suitable for the invention as long as it is suitable for pharmaceutical topical application. The pore size is given by the manufacturer.

Further, it is required, in order to produce a pouch, that the margins of the semipermeable and the impermeable membrane can be tightly sealed together by heat or other techniques which are not harmful for the enclosed bacteria. Sealing of the two membranes can, as an example, be carried out with a commercially available film sealing device with temperature control such as "Fermant 22" (joke renotec, Bergisch Gladbach, Germany) at temperatures of 170 to 280 °C, preferably 230 to 275 °C. Other conventional sealing methods are equally suitable

The semipermeable membrane according to the invention is preferably but not exclusively made from polycarbonate. Any other polymer fulfilling the criteria mentioned before can also be used in the invention. Suitable polymers for the semipermeable membrane are polyethersulfone, polysulfone cellulose, cellulosic esters (e.g. cellulose acetate, cellulose nitrate), silicones, polyamides, polyamide imide, polyamide urea, polyacryl nitrile, polyethylene, polypropylene, polytetrafluorethylene, polyvinylidene fluoride, polyvinyl chloride, polypiperazine amide. Combinations of these polymers layered in at least two thin films are also suitable.

For the water-impermeable membrane, any polymer material is suitable which cannot be permeated by liquid water and which can be sealed, e.g. by heat, with the semipermeable membrane as described above. Preferably, for better sealability with the semipermeable membrane, the same polymer type is used. Particularly preferred is polycarbonate.

The two membranes in the desired shape and size are sealed as explained above at three sides before the insert is introduced (see below), thus forming a pouch.

The thickness of the polymer materials can be appropriately selected. Usually, the water-impermeable membrane has a thickness of 0.01 to 0.10 mm, preferably 0.015 to 0.025 mm and the semipermeable membrane may have a thickness of 0.002 to 0.02 mm, preferably 0.075 mm.

A material suitable as insert in pharmaceutical plasters, such as a commercially available nonwoven, is then inserted into the pouch. As material for the insert, any material which does not interfere with the probiotics can be used. Examples are nonwovens made of viscose or polypropylene, polyesters, polyamides and mixtures thereof. Further, commonly known polyurethane foams are suitable as insert material. The insert materials usually have a high water-absorption such as 500 to 2200 g/m², preferably 920 to 980 g/m² in order to be able to absorb the probiotic suspension.

The pouch with the insert is then heat-sterilized, e.g. by autoclaving at 121 °C for 20 min.

The probiotic is subsequently applied to the insert within the pouch in form of a suspension. Preferably, the same culture medium such as MRS broth used for the initial cultivation is used as suspension liquid. Suitable protectants such as trehalose can be added to the suspension. The suspension is adjusted so that it contains 1.5 to 4.5 × 10¹⁰ CFU probiotic bacteria per ml, preferably 2 to 4 x 10¹⁰ CFU/ml, most preferably 3 × 10¹⁰ CFU/ml.

CFU means "colony forming unit" and is determined manually in this invention by a standard method (using a pen and a click-counter).

The probiotics used according to the invention are any probiotics having antimicrobial activity against skin pathogens and having GRAS status, and having a beneficial effect on infectious skin disorders, such as various *Lactobacillus sp., Bifidobacterium* sp., and Streptococci such as those mentioned above in the part "Background art". Preferably, probiotics of the order *Lactobacillales, Lactobacillus* sp., *Streptococcus salivarius* K12, *Streptococcus salivarius* M18 and/or *Bifidobacterium* sp. are used according to the invention. A particularly preferable *Lactobacillus* species used according to the invention is *L. plantarum,* and the most preferable strain is *L. plantarum* 8P-A3 (DSM 32835). *S. salivarius* K12 is a strain used in commercially available probiotic preparations BLIS K12^{™} probiotic powder. *S. salivarius* M18 is a strain contained in commercially available probiotic dental care products such as ProBio-Dent, Life Extension FLORASSIST, Bio-Kick M18 Zahn. K12 and M18 are strains which have been found by Professor John Tagg of the Microbiology Department at the University of Otago and later-on developed to probiotic nutrition supplements by the company BLIS Technologies, Dunedin, New Zealand.

Mixtures of two or more of the probiotics can be used, although the most preferable embodiment of the invention is a formulation containing *L. plantarum* 8P-A3 as single probiotic.

*GRAS status* is defined as "Generally Recognized As Safe", i.e. the respective strains are authorized by the FDA and not limited in daily intake according to the Federal Food, Drug, and Cosmetic Act (FFDAC).

The thus-obtained pouch is then preferably incubated, e.g. for 15 min at 2 to 6 °C, preferably 4 °C.

The pouch is then dried under usual conditions known to the skilled person, e.g. under vacuum of 100 to 1500 Pa (1-15 mbar) at 5 to 25 °C for 24 h or by rapid freezing at temperatures ≤80°C and subsequent freeze drying (lyophilisation).

The fourth (still open) side of the pouch is finally sealed as described above, if desired.

The formulation contains about 10⁶ to 10¹², preferably 10⁸ to 10¹⁰ CFU probiotic bacteria per cm² of the (preferably nonwoven) polymer insert surface.

The obtained formulation is designated as patch. It can be applied as such onto the skin.

It can be fixed using a usual plaster or pharmaceutically acceptable adhesive tape or by a bandage, if desired.

The shape and size of the patch can be varied according to the desired area of application. Such different shapes are well known. In general, the shape is a square or a rectangle, but round, oval or butterfly-like shapes are also applicable. Other shapes, also irregular ones, are also within the scope of the invention.

The formulation of the invention is applied to the naked skin or onto wounds so that the "inner side" - the semipermeable membrane - is in contact with the area to be treated. In this way, diffusible antimicrobial products (i.e. metabolites) produced by the probiotics (but not the bacteria / probiotics themselves) can reach the skin. The antimicrobial metabolites exert the pharmaceutical effect of the inventive formulation on the diseased skin, whereas the probiotic bacteria do not get into contact therewith.

The thus-obtained patch can further be supplemented with common means for fixation at the skin, such as an adhesive tape which is commonly used in plasters. The adhesive tape can be provided in a manner that it covers the complete surface of the patch, or it can be provided only on at least one of the margins. The adhesive side can be protected by a commonly known release film which is removed before applying the formulation onto the skin.

Further, a commonly known fibre material or a net-like material such as gauze which functions as protective layer can be provided between the water-impermeable (upper) side of the patch and the adhesive tape.

The thus obtained formulation is designated as plaster or bandage. It can also be designated as transdermal therapeutic system of the reservoir type. Although the mechanism of action is not transdermal in the strict sense, the structure of the plaster is similar to usual transdermal therapeutic patches and thus this designation is used for the inventive formulation.

The number of patches and/or plasters which are applied to the diseased skin is selected appropriately. At least one plaster or patch is applied, but the number can be larger depending on the area to be covered and the different sites which need treatment. For example, one plaster (or one patch) can be applied on the forehead, one on the chin, one or more on each cheek, respectively, one on the nose and/or one on other parts of the skin if acne is to be treated on the complete face.

The patch(es)/plaster(s) can remain on the skin for an appropriate time of up to 24 h, and then the patch(s)/plaster(s) can be removed and replaced by fresh patch(es)/plasters, if needed.

In a suitable way they can be applied intermittently only during the night, which may enhance patient compliance in particular if the application is on the face, whereas during the day the skin is left as it is or treated with common cosmetic or pharmaceutical formulations such as creams, lotions or the like, respectively.

A commonly known hydrogel, such as a commercially available hydrogel, can be applied onto the skin immediately prior to the application of the patch(es) or plaster(s) in order to accomplish a tight contact to the skin surface.

A fiber material for absorption of wound exudate such as sterile gauze can additionally or alternatively be applied between the inventive formulation and the diseased skin, if needed.

The probiotic bacteria within the formulation are preserved in a dormant, dried state to keep the formulation stable for at least 6 months to be applied to the skin or wounds.

"Stable" means that after 6 months of storage under ambient conditions at least 2 % of the bacteria in the formulation are still viable and are able to multiply and exert antimicrobial activity against skin pathogens.

"Viable" is defined as the ability to multiply via binary fission under the controlled conditions. This can be determined by *in-vitro* culturing in liquid media and under culture conditions suitable for the respective probiotic bacterial strain.

Appropriate known protectants and protective packaging provide the required stability. Depending on the probiotic in the patch the protectants to be applied in suitable amounts are for example, but not exclusively: (I) disaccharides like trehalose, sucrose, maltose, lactose; (II) polyols like sorbitol and mannitol; (III) oligosaccharides and polysaccharides like galacto-oligosaccharides, inulin, starch, and maltodextrins; (IV) amino acids and peptides like glutamic acid and glutathione; (V) other like ascorbic acid, ectoine and hydroxyectoine. They may be used either as single compound or in combination of two or more compounds.

The invention provides a formulation which is activated when applied onto diseased skin, whereby water and other substances from the skin surface diffuse into the pouch to induce metabolism, growth, and production of antimicrobial substances of the enclosed probiotics.

The thus-reactivated formulation can deliver antimicrobially active substances (in particular the metabolites produced by the probiotic) onto the skin by diffusion through the semipermeable membrane. It is active by inhibition of growth and killing of human skin pathogens, for example of the taxonomic units *Staphylococcus* including S. *aureus* (also methicillin-resistant [MRSA]), *Streptococcus* including *S*. *pyogenes* and *S*. *dysgalactiae, Enterobacteriaceae* including *Escherichia coli* and *Klebsiella pneumoniae, Pseudomonas aeruginosa,* and *Cutibacterium (Propionibacterium*) *acnes.* Its activity against other skin pathogens is not excluded. In particular, the inventive formulation exerts its activity against *Cutibacterium (Propionibacterium) acnes, Staphylococcus aureus, Streptococcus pyogenes, Streptococcus dysgalactiae, Escherichia coli, Klebsiella pneumoniae,* and *Pseudomonas aeruginosa.*

By applying the inventive formulation, the incidence and/or severity of a skin disorder is reduced accordingly.

The antimicrobial activity of the composition allows its application to treat - but not exclusively - the following skin disorders (or at least support their treatment): acne vulgaris, infected burn wounds, infected venous leg ulcers, superinfected lesions in atopic dermatitis patients. Other skin disorders might be positively influenced.

The application of the composition also is expected to have a cosmetic effect on normal skin, in particular of the face. For example, it is expected that wrinkles and/or fine lines can be reduced, hydration of the skin can be improved, thereby reducing a dry feeling, and/or a luminous complexion can be achieved.

In a special embodiment, the *Lactobacillus plantarum,* strain 8P-A3 (Tsapieva, A. et al., 2011), or another *Lactobacillus* strain having at least 97% sequence similarity with SEQ ID No 1 in its 16S rRNA gene is used in the topical formulation according to the invention.

Sequence similarity is ascertained by determining the nucleotide sequence of the 16S RNA gene of the strain by an established sequencing method with low error rate, e g. the Sanger (chain termination) method and alignment of the respective sequences with NCBI BLAST.

*Biologically pure* is used in the present invention according to the common understanding of the skilled person. The term means that besides the strain of interest no other bacteria, viruses or other microorganisms are detectable.

### Brief Description of the Drawing

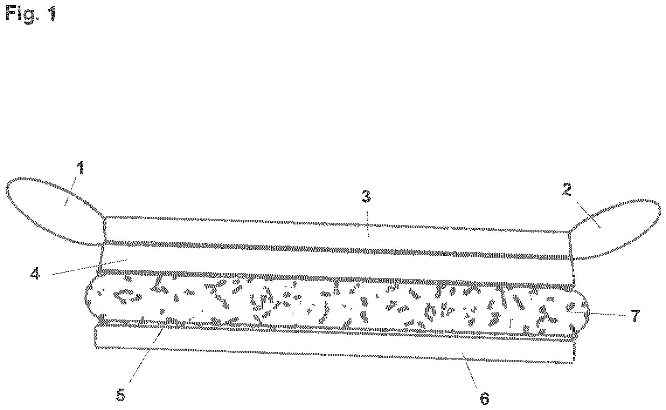

Fig. 1 shows a section of an example of a plaster according to the invention comprising a semipermeable membrane (5), a composition comprising the probiotic bacteria (7), a water-impermeable membrane (4), fixation means (1, 2) and a protective layer (3). Further, it shows a hydrogel or a material for absorption of wound exudate (6) placed between the plaster and the diseased skin.

It is emphasised that the specifics shown in the figure are by way of example and for purposes of illustrative discussion of the different embodiments of the present invention only. The drawing is presented in the cause of providing what is believed to be the most useful and ready description of the principles and conceptual aspects of the invention. In this regard no attempt is made to show structural details of the invention in more detail than is necessary for a fundamental understanding of the invention. The legend of the drawing discloses to those skilled in the art how the different forms of the invention may be embodied in practice.

### EXAMPLES

The following examples are provided as a pure illustration of the present invention and are not intended to be considered as limiting the protection scope as it appears from the appended claims.

### Example 1

### Topical application of the composition ("Probiotic Patch") for the treatment of acne vulgaris and other skin disorders (co-)caused by pathogenic bacteria

Two commercially available polycarbonate membranes, one water-impermeable (0.02 mm thick, Makrofol^{®}, Covestro, Leverkusen, Germany) and one semipermeable (thickness: 5-10 µm, pore size: 0.2 µm, Nuclepore^{®}, Whatman, received through Reichelt Chemietechnik GmbH + Co., Heidelberg, Germany), each approximately 4 cm by 4 cm, were heat-sealed at three sides at 170 °C using a commercially available programmable film sealing device ("Fermant 22", joke renotec, Bergisch Gladbach, Germany).

An insert of a commercially available nonwoven material (2.0 cm by 2.0 cm; fibre composition: viscose, polypropylene; weight: 114 g/cm²; thickness: 1.4 mm; water absorption: 950 g/m², M1556, Freudenberg New Technologies KG, Weinheim, Germany) was put into the obtained membrane pouch sealed at three sides. This formulation of the polycarbonate membranes and the nonwoven insert was heat-sterilized by autoclaving at 121°C for 20 minutes in a standardized procedure (DIN EN ISO 17665-1:2006).

The *Lactobacillus plantarum* strain 8P-A3 (DSM 32835) was grown in MRS-broth to the early stationary phase, harvested by centrifugation and resuspended in 0.1 of the original volume in fresh MRS medium containing 5% trehalose so that the suspension contained approximately 3 x 10¹⁰ CFU of probiotic bacteria per ml.

After equilibration of the *L*. *plantarum* suspension at room temperature for 1 hour, 200 µl of the suspension was pipetted into the welded pouches, incubated for 15 min. at 4°C, and those were immediately dried in a vacuum drying chamber under the following conditions: drying time: 24 hours, chamber temperature: approximately 22-25 °C, final chamber pressure: 100-1500 Pa (1-15 mbar).

Before the following *in-vitro* testing for antibacterial activity of the dried pouches - for some experiments - they were heat-sealed at the fourth side (not yet sealed before). Some experiments were carried out with patches which were left open at the fourth side. Both types of patches showed identical effects.

*In-vitro* effects of the dried pouches containing the probiotic *L. plantarum* strain DSM 32835: The antimicrobial activity of the sealed pouches was tested in-vitro against the following pathogens as described in the following:

| **Species** | **Designation** | **Strain Collection (Website)** |
|---|---|---|
| *Cutibacterium acnes* | DSM-1897 | DSMZ.de |
| *Pseudomonas aeruginosa* | DSM-1117 | DSMZ.de |
| *Staphylococcus aureus* | DSM-799 | DSMZ.de |

Pouches containing MRS medium with 5% trehalose but no probiotic bacteria and vacuum-dried as above served as controls.

For these experiments the pathogens *C. acnes* and *S. aureus* were incorporated into Wilkins-Chalgren (WC) agar or Brain Heart Infusion (BHI) agar, respectively, with an approximate cell count of 10³ per ml. The dried *L*. *plantarum-*containing patches obtained as described above (after storage for four days at room temperature) were applied onto the surface of the agar plates containing the pathogens. Then these agar plates were incubated anaerobically at 33 °C for two days (*S*. *aureus*) and six days (*C. acnes*)*,* respectively.

For *P. aeruginosa* as a target bacterium, a "deferred antagonism" test was performed as follows:
The dried patches were put onto BHI agar plates and those were incubated anaerobically at 33 °C for two days. After that the patches were removed and the indicator *P. aeruginosa* strain (~ 10⁵ CFU/mL) was streaked in two perpendicular lines across the surface area where the patches had been placed before the incubation for two days and been removed after. These test plates were incubated aerobically at 33 °C for 18 hours.

Inhibitory activity of the *L*. *plantarum* probiotic-containing patches applied to agar plates: The incubation of the probiotic-containing patches for two days on agar plates under the conditions described above led to a complete (under the whole surface of the patch) *in-vitro* inhibition of the pathogens *C*. *acnes, S. aureus,* and *P. aeruginosa.*

Analogically to the *in-vitro* testing of antibiotics, this indicates an *in-vivo* activity of the composition when applied to body surfaces, especially, but not exclusively, the human skin.

### Example 2

### Topical application of the composition ("Probiotic Patch") for the treatment of acne vulgaris and other skin disorders (co-)caused by Staphylococcus aureus

In the same manner as in Example 1, patches were obtained using *Streptococcus salivarius* strain K12 as probiotic.

The *Streptococcus salivarius* strain K12 (isolated from BLIS K12^{™}, probiotic powder) was grown in mWC (modified Wilkins-Chalgren) broth to the early stationary phase, harvested by centrifugation and resuspended in 0.1 of the original volume in fresh mWC medium containing 5% trehalose so that the suspension contained approximately 10⁹ CFU of probiotic bacteria per ml.

The pouches were obtained and dried as in Example 1.

*In-vitro* effects of the dried pouches containing the probiotic *S. salivarius* strain K12:
The antimicrobial activity of the sealed pouches was tested in-vitro against the following pathogen as described in the following:

| **Species** | **Designation** | **Strain Collection (Website)** |
|---|---|---|
| *Staphylococcus aureus* | DSM-799 | DSMZ.de |

Pouches containing mWC medium with 5% trehalose but no probiotic bacteria and vacuum-dried as above served as controls.

For these experiments the pathogen *S. aureus* was incorporated into Brain Heart Infusion (BHI) agar with an approximate cell count of 10³ per ml. The dried *S. salivarius-containing* patches obtained as described above (after storage for four days at room temperature) were applied onto the surface of the agar plates containing the pathogen. Then these agar plates were incubated anaerobically at 33 °C for two days.

Inhibitory activity of the *S. salivarius* K12 probiotic-containing patches applied to agar plates: The incubation of the probiotic-containing patches for two days on agar plates under the conditions described above led to a complete (under the whole surface of the patch) *in-vitro* inhibition of the pathogen *S. aureus.*

This inhibition was also achieved after 3 months storage at room temperature of the dried *S. salivarius*-containing patches obtained as described above.

An inhibition of this kind and strength could also be observed when one drop of a sterile hydrogel (-50 µl, "DracoHydrogel", Dr. Ausbüttel & Co. GmbH, Witten, Germany) was placed between the patches and the test agar plate containing *S. aureus.*

Analogically to the *in-vitro* testing of antibiotics, this indicates an *in-vivo* activity of the composition when applied to body surfaces, especially, but not exclusively, the human skin.

### Example 3

### Topical application of the composition ("Probiotic Patch") for the treatment of skin disorders like venous leg ulcers or infected burn wounds caused by Escherichia coli, P. aeruginosa or Staphylococcus aureus

In the same manner as in Example 2, patches were obtained using *Streptococcus salivarius* strain M18 as probiotic bacterium.

The *Streptococcus salivarius* strain M18 (isolated from PROBIO-DENT^{®}, tablets, SYXYL GmbH & Co. KG, Cologne, Germany) was grown in mWC (modified Wilkins-Chalgren) broth to the early stationary phase, harvested by centrifugation and resuspended in 0.1 of the original volume in fresh mWC medium containing 5% trehalose so that the suspension contained approximately 10⁹ CFU of probiotic bacteria per ml.

The pouches were obtained and dried as in Examples 1 and 2.

*In-vitro* effects of the dried pouches containing the probiotic *S. salivarius* strain M18:
The antimicrobial activity of the sealed pouches was tested in-vitro against the following pathogens as described in the following:

| **Species** | **Designation** | **Strain Collection (Website)** |
|---|---|---|
| *Escherichia coli* | DSM-1103 | DSMZ.de |
| *Pseudomonas aeruginosa* | DSM-1117 | DSMZ.de |
| *Staphylococcus aureus* | DSM-799 | DSMZ.de |

Pouches containing mWC medium with 5% trehalose but no probiotic bacteria and vacuum-dried as above served as controls.

For the experiment with the pathogen *S. aureus* as target bacterium, this was incorporated into Brain Heart Infusion (BHI) agar with an approximate cell count of 10³ per ml. The dried *S. salivarius*-containing patches obtained as described above (after storage for four days at room temperature) were applied onto the surface of the agar plates containing the pathogen. Then these agar plates were incubated anaerobically at 33 °C for two days.

For *E. coli* DSM-1103 and *P. aeruginosa* DSM-1117 as target bacteria, a "deferred antagonism" test was performed as follows:
The dried patches were put onto BHI agar plates and those were incubated anaerobically at 33 °C for two days. After that the patches were removed and the indicator *E. coli* and *P*. *aeruginosa* strain, respectively (~ 10⁵ CFU/mL), were streaked in two perpendicular lines across the surface area where the patches had been placed before the incubation for two days and been removed after. These test plates were incubated aerobically at 33 °C for 18 hours.

Inhibitory activity of the *S. salivarius* M18 probiotic-containing patches applied to agar plates: The incubation of the probiotic-containing patches for two days on agar plates under the conditions described above led to a complete (under the whole surface of the patch) *in-vitro* inhibition of the pathogens *E. coli* DSM-1103, *P. aeruginosa* DSM-1117, and *S. aureus* DSM-799.

An inhibition of this kind and strength could also be observed when one drop of a sterile hydrogel (-50 µl, "DracoHydrogel", Dr. Ausbüttel & Co. GmbH, Witten, Germany) was placed between the patches and the test agar plate with the *S. aureus* strain incorporated (test not done in the deferred antagonism tests with *E. coli* or *P. aeruginosa* as target bacteria).

Analogically to the *in-vitro* testing of antibiotics, this indicates an *in-vivo* activity of the composition when applied to body surfaces, especially, but not exclusively, the human skin.

### Reference to deposited biological material

The *Lactobacillus plantarum* strain 8P-A3, used in particular embodiments of the invention, was deposited by the assignee under the terms of the Budapest Treaty with Leibniz-lnstitut DSMZ-Deutsche Sammlung von Mikroorganismen und Zellkulturen GmbH, Braunschweig, Germany, on 14 June 2018 and assigned Accession No. DSM 32835.

### List of reference signs

- 1, 2: fixation means
- 3: protective layer
- 4: water-impermeable membrane
- 5: semipermeable membrane
- 6: hydrogel or material for absorption of wound exudate
- 7: enclosed probiotic bacteria

## Claims

1. A topical pharmaceutical or cosmetic formulation comprising two polymer membranes (4,5) forming a pouch, the membranes enclosing probiotic bacteria (7) having antimicrobial activities against skin pathogens sandwiched therebetween, one of the membranes being semipermeable, which allows water and other substances from the skin to diffuse into the pouch and metabolic products of the enclosed probiotic bacteira to diffuse through it, and having a pore size which does not allow penetration of the bacteria, and the second membrane being water-impermeable.

2. A topical formulation according to claim 1, additionally comprising an insert between the two membranes, the formulation containing from about 10⁸ to 10¹⁰ CFU probiotic bacteria per cm² of the insert surface.

3. A topical formulation of claim 1 or 2 which is in the form of a patch or plaster.

4. A topical formulation of any of claims 1 to 3 wherein the probiotic bacteria are of the order *Lactobacillales.*

5. A topical pharmaceutical formulation according to any of the preceding claims for use in a method for treating a skin disorder, wherein the skin disorder is a skin or wound infection.

6. The topical pharmaceutical formulation for the use according to claim 5,
wherein the skin disorder is one or more of acne or superinfected acne lesions, infected burn wound, venous leg ulcer, and infected atopic dermatitis lesions.

7. The topical pharmaceutical formulation for the use according to claims 5 or 6, wherein the skin disorder is caused at least in part by one or more of *Cutibacterium (Propionibacterium)* species, *Staphylococcus* species, *Streptococcus* species, *Enterobacteriaceae* genera and species, and *Pseudomonas* species.

8. A pharmaceutical or cosmetic topical formulation according to any of claims 1 to 4, or a topical pharmaceutical formulation for use according to claims 5 to 7, comprising a biologically pure culture of *Lactobacillus plantarum* strain 8P-A3 on deposit at Leibniz Institute DSMZ-German Collection of Microorganisms and Cell Cultures, Braunschweig, Germany, under accession number DSM 32835, **and/or**
a *Lactobacillus* strain having at least 97% sequence similarity with the sequence shown in SEQ ID NO: 1 in its 16S rRNA gene.

9. A topical pharmaceutical or cosmetic formulation according to any of claims 1 to 4, or a topical pharmaceutical formulation for use according to claims 5 to 8, additionally comprising protectant substances to preserve long term viability of the enclosed probiotic bacteria.

10. The *Lactobacillus plantarum* strain 8P-A3 having the accession number DSM 32835 or a *Lactobacillus* strain having at least 97% sequence similarity with the sequence shown in SEQ ID NO: 1 in its 16S rRNA gene for use in a method of topically treating infectious skin disorders by the topical formulation of any of claims 1 to 9.

11. The *L. plantarum* strain 8P-A3 having the accession number DSM 32835 or a *Lactobacillus* strain having at least 97% sequence similarity with the sequence shown in SEQ ID NO: 1 in its 16S rRNA gene
for the use defined in claim 10, wherein the infectious skin disorder is one or more of acne vulgaris, infected lesions of venous leg ulcers, infected burn wounds, and infected skin lesions of atopic dermatitis patients.

12. The *L. plantarum* strain 8P-A3 having the accession number DSM 32835 or a *Lactobacillus* strain having at least 97% sequence similarity with the sequence shown in SEQ ID NO: 1 in its 16S rRNA gene
for the use defined in claims 10 or 11, wherein the infectious skin disorder is caused at least in part by one or more of *Cutibacterium (Propionibacterium)* species, *Staphylococcus* species, *Streptococcus* species, *Enterobacteriaceae* genera and species, and *Pseudomonas* species.

13. Cosmetic use of a formulation as defined in any of claims 1 to 4 for improving the appearance of the human skin, reducing fine lines and/or wrinkles, hydrating the skin and/or achieving a luminous complexion.

14. Cosmetic use of a formulation as defined in claim 13, wherein the formulation comprises a biologically pure culture of *Lactobacillus plantarum* strain 8P-A3 on deposit at Leibniz Institute DSMZ-German Collection of Microorganisms and Cell Cultures, Braunschweig, Germany, under accession number DSM 32835, **and/or** a *Lactobacillus* strain having at least 97% sequence similarity with the sequence shown in SEQ ID NO: 1 in its 16S rRNA gene.

## Patentansprüche

1. Topische pharmazeutische oder kosmetische Formulierung, umfassend zwei Polymermembranen (4, 5), die eine Tasche bilden, wobei die Membranen sandwichartig dazwischen eingeschlossene probiotische Bakterien (7) mit antimikrobiellen Aktivitäten gegen Hautpathogene enthalten, wobei eine der Membranen semipermeabel ist, die es Wasser und anderen Substanzen aus der Haut erlaubt, in den Beutel zu diffundieren, und die es Stoffwechselprodukten der eingeschlossenen probiotischen Bakterien erlaubt, durch sie hindurchzudiffundieren, und die eine Porengröße hat, die das Eindringen der Bakterien nicht erlaubt, und die zweite Membran wasserundurchlässig ist.

2. Topische Formulierung nach Anspruch 1, die zusätzlich eine Einlage zwischen den beiden Membranen umfasst, wobei die Formulierung etwa 10⁸ bis 10¹⁰ CFU probiotische Bakterien pro cm² der Einlagenoberfläche enthält.

3. Topische Formulierung nach Anspruch 1 oder 2, die in Form eines Pflasters vorliegt.

4. Topische Formulierung nach einem der Ansprüche 1 bis 3, wobei die probiotischen Bakterien aus der Ordnung der *Lactobacillales* stammen.

5. Topische pharmazeutische Formulierung nach einem der vorangehenden Ansprüche zur Verwendung in einem Verfahren zur Behandlung einer Hauterkrankung, wobei die Hauterkrankung eine Haut- oder Wundinfektion ist.

6. Topische pharmazeutische Formulierung für die Verwendung nach Anspruch 5, wobei die Hauterkrankung eine oder mehrere von Akne oder superinfizierten Akneläsionen, infizierten Verbrennungswunden, venösem Beingeschwür, und infizierten atopische Dermatitisläsionen ist.

7. Topische pharmazeutische Formulierung für die Verwendung nach den Ansprüchen 5 oder 6, wobei die Hauterkrankung zumindest teilweise durch eine oder mehrere von *Cutibacterium* (*Propionibacterium*)-Spezies*, Staphylococcus-Spezies, Streptococcus-Spezies, Enterobacteriaceae-*Gattungen und -Spezies und Pseudomonas-Spezies verursacht wird.

8. Pharmazeutische oder kosmetische topische Formulierung nach einem der Ansprüche 1 bis 4 oder topische pharmazeutische Formulierung zur Verwendung nach einem der Ansprüche 5 bis 7, umfassend eine biologisch reine Kultur von *Lactobacillus plantarum* Stamm 8P-A3, hinterlegt beim Leibniz-Institut DSMZ-Deutsche Sammlung von Mikroorganismen und Zellkulturen, Braunschweig, Deutschland, unter der Zugangsnummer DSM 32835, **und/oder**
einen *Lactobacillus-Stamm* mit mindestens 97 % Sequenzähnlichkeit mit der in SEQ ID NO: 1 gezeigten Sequenz in seinem 16S rRNA-Gen.

9. Topische pharmazeutische oder kosmetische Formulierung nach einem der Ansprüche 1 bis 4 oder topische pharmazeutische Formulierung zur Verwendung nach einem der Ansprüche 5 bis 8, die zusätzlich Schutzsubstanzen zur Erhaltung der langfristigen Lebensfähigkeit der eingeschlossenen probiotischen Bakterien enthält.

10. *Lactobacillus plantarum*-Stamm 8P-A3 mit der Hinterlegungsnummer DSM 32835 oder *Lactobacillus-Stamm* mit mindestens 97% Sequenzähnlichkeit mit der in SEQ ID NO: 1 gezeigten Sequenz in seinem 16S rRNA-Gen zur Verwendung in einem Verfahren zur topischen Behandlung infektiöser Hauterkrankungen durch die topische Formulierung nach einem der Ansprüche 1 bis 9.

11. *L. plantarum-Stamm* 8P-A3 mit der Hinterlegungsnummer DSM 32835 oder *Lactobacillus-Stamm* mit mindestens 97% Sequenzähnlichkeit mit der in SEQ ID NO: 1 gezeigten Sequenz in seinem 16S rRNA-Gen für die in Anspruch 10 definierte Verwendung, wobei die infektiöse Hauterkrankung eine oder mehrere von Akne vulgaris, infizierten Läsionen von venösen Beingeschwüren, infizierten Verbrennungswunden und infizierten Hautläsionen von Patienten mit atopischer Dermatitis ist.

12. *L. plantarum-Stamm* 8P-A3 mit der Hinterlegungsnummer DSM 32835 oder *Lactobacillus-Stamm* mit mindestens 97% Sequenzähnlichkeit mit der in SEQ ID NO: 1 gezeigten Sequenz in seinem 16S rRNA-Gen für die in den Ansprüchen 10 oder 11 definierte Verwendung, wobei die infektiöse Hauterkrankung zumindest teilweise durch eine oder mehr von *Cutibacterium (Propionibacterium)-Spezies, Staphylococcus-Spezies, Streptococcus-*Spezies, Enterobacteriaceae-Gattungen und -Spezies und *Pseudomonas-*Spezies verursacht wird.

13. Kosmetische Verwendung einer in einem der Ansprüche 1 bis 4 definierten Formulierung zur Verbesserung des Aussehens der menschlichen Haut, zur Reduzierung feiner Linien und/oder Falten, zur Hydratisierung der Haut und/oder zur Erzielung eines strahlenden Teints.

14. Kosmetische Verwendung einer Formulierung wie in Anspruch 13 definiert, wobei die Formulierung eine biologisch reine Kultur des *Lactobacillus plantarum-Stammes* 8P-A3, hinterlegt beim Leibniz-Institut DSMZ-Deutsche Sammlung von Mikroorganismen und Zellkulturen, Braunschweig, Deutschland, unter der Hinterlegungsnummer DSM 32835, umfasst, **und/oder** einen *Lactobacillus-Stamm* mit mindestens 97% Sequenzähnlichkeit mit der in SEQ ID NO: 1 gezeigten Sequenz in seinem 16S rRNA-Gen.

## Revendications

1. Formulation pharmaceutique ou cosmétique topique comprenant deux membranes polymères (4, 5) formant une poche, les membranes renfermant des bactéries probiotiques (7) ayant des activités antimicrobiennes contre les agents pathogènes de la peau prises en sandwich entre elles, l'une des membranes étant semi-perméable, qui permet à l'eau et à d'autres substances de la peau de diffuser dans la poche et aux produits métaboliques des bactéries probiotiques enfermées de diffuser à travers elle, et ayant une taille de pores qui ne permet pas la pénétration des bactéries, et la seconde membrane étant imperméable à l'eau.

2. Formulation topique selon la revendication 1, comprenant en outre un insert entre les deux membranes, la formulation contenant d'environ 10⁸ à 10¹⁰ UFC de bactéries probiotiques par cm² de la surface de l'insert.

3. Formulation topique selon la revendication 1 ou 2 qui se présente sous la forme d'un patch ou d'un emplâtre.

4. Formulation topique selon l'une quelconque des revendications 1 à 3, dans laquelle les bactéries probiotiques sont de l'ordre des *Lactobacillales.*

5. Formulation pharmaceutique topique selon l'une quelconque des revendications précédentes pour une utilisation dans une méthode de traitement d'un trouble cutané, dans laquelle le trouble cutané est une infection de la peau ou d'une plaie.

6. Formulation pharmaceutique topique pour l'utilisation selon la revendication 5, dans laquelle le trouble cutané est une ou plusieurs des lésions d'acné ou d'acné surinfectée, une plaie de brûlure infectée, un ulcère veineux de jambe, et des lésions de dermatite atopique infectées.

7. Formulation pharmaceutique topique pour l'utilisation selon les revendications 5 ou 6, dans laquelle le trouble cutané est causé au moins en partie par une ou plusieurs des espèces *Cutibacterium (Propionibacterium),* des espèces *Staphylococcus,* des espèces *Streptococcus,* des genres et espèces *Enterobacteriaceae,* et des espèces *Pseudomonas.*

8. Formulation topique pharmaceutique ou cosmétique selon l'une quelconque des revendications 1 à 4, ou formulation pharmaceutique topique à utiliser selon les revendications 5 à 7, comprenant une culture biologiquement pure de la souche 8P-A3 de *Lactobacillus plantarum* en dépôt à l'Institut Leibniz DSMZ-Collection allemande de micro-organismes et de cultures cellulaires, Braunschweig, Allemagne, sous le numéro d'accession DSM 32835, **et/ou** une souche de *Lactobacillus* ayant une similarité de séquence d'au moins 97% avec la séquence montrée dans SEQ ID NO : 1 dans son gène 16S rRNA.

9. Formulation pharmaceutique ou cosmétique topique selon l'une quelconque des revendications 1 à 4, ou formulation pharmaceutique topique à utiliser selon les revendications 5 à 8, comprenant en outre des substances protectrices pour préserver la viabilité à long terme des bactéries probiotiques incluses.

10. La souche 8P-A3 de *Lactobacillus plantarum* ayant le numéro d'accession DSM 32835 ou une souche de *Lactobacillus* ayant une similarité de séquence d'au moins 97% avec la séquence montrée dans SEQ ID NO : 1 dans son gène 16S rRNA pour une utilisation dans une méthode de traitement topique des troubles infectieux de la peau par la formulation topique de l'une quelconque des revendications 1 à 9.

11. Souche 8P-A3 de *L. plantarum* ayant le numéro d'accession DSM 32835 ou souche de *Lactobacillus* ayant une similarité de séquence d'au moins 97% avec la séquence montrée dans SEQ ID NO : 1 dans son gène 16S rRNA pour l'utilisation définie dans la revendication 10, dans laquelle l'affection cutanée infectieuse est une ou plusieurs des affections suivantes : acné vulgaire, lésions infectées d'ulcères veineux de jambe, plaies de brûlure infectées et lésions cutanées infectées de patients atteints de dermatite atopique.

12. La souche 8P-A3 de *L. plantarum* ayant le numéro d'accession DSM 32835 ou une souche de *Lactobacillus* ayant une similarité de séquence d'au moins 97% avec la séquence montrée dans SEQ ID NO : 1 dans son gène 16S rRNA pour l'utilisation définie dans les revendications 10 ou 11, dans laquelle le trouble cutané infectieux est causé au moins en partie par une ou plusieurs des espèces *Cutibacterium (Propionibacterium),* des espèces *Staphylococcus,* des espèces *Streptococcus,* des genres et espèces *Enterobacteriaceae,* et des espèces *Pseudomonas.*

13. Utilisation cosmétique d'une formulation telle que définie dans l'une quelconque des revendications 1 à 4 pour améliorer l'aspect de la peau humaine, réduire les ridules et/ou les rides, hydrater la peau et/ou obtenir un teint lumineux.

14. Utilisation cosmétique d'une formulation telle que définie dans la revendication 13, dans laquelle la formulation comprend une culture biologiquement pure de la souche 8P-A3 de *Lactobacillus plantarum* en dépôt à l'Institut Leibniz DSMZ-Collection allemande de micro-organismes et de cultures cellulaires, Braunschweig, Allemagne, sous le numéro d'accession DSM 32835, **et/ou** une souche de Lactobacillus ayant une similarité de séquence d'au moins 97 % avec la séquence présentée dans la SEQ ID NO : 1 dans son gène 16S rRNA.
